# FASCICULE DE BREVET EUROPEEN

(11) **EP 0 689 867 B1**
(45) Date de publication et mention de la délivrance du brevet: **11.11.1998**
(21) Numéro de dépôt: 95401266.2
(22) Date de dépôt: 31.05.1995
(51) Int. Cl.: B01J 2/02, A61J 3/02, A61K 9/16, C09B 67/00

(54) **Procédé d'obtention d'une matière première sous forme de granulés**
Verfahren zur Herstellung von Rohstoffen in Granulatform
Process for obtaining raw materials in granular form

(30) Priorité: 03.06.1994 FR 9406843
(43) Date de publication de la demande: 03.01.1996
(73) Titulaire: L'OREAL, 75008 Paris (FR)
(72) Inventeur: Gurfein, Véronique, F-92350 Le Plessis Robinson (FR); Zaffran, Christian, F-78310 Elancourt (FR); Lapoirie, Eric, F-93250 Villemonble (FR)
(74) Mandataire: Casalonga, Axel

(56) Documents cités:
- EP-A- 0 150 158
- WO-A-90/13285
- US-A- 3 928 566
- DATABASE WPI Week 8319 Derwent Publications Ltd., London, GB; AN 83-45684K & JP-A-58 056 669 (OSAKA GAS KK) , 4 Avril 1983
- PATENT ABSTRACTS OF JAPAN vol. 13 no. 402 (C-633) ,6 Septembre 1989 & JP-A-01 146901 (KURITA WATER IND LTD) 8 Juin 1989,

## Description

La présente invention concerne un procédé d'obtention d'une matière première anhydre sous forme de granulés, microporeux, et une matière première anhydre sous forme de granulés, microporeux.

L'invention concerne également l'utilisation de granulés anhydres de matière première dans le domaine cosmétique, capillaire, vétérinaire ou pharmaceutique.

Les matières premières sous forme pulvérulente (poudre), telles que les matières colorantes, présentent divers inconvénients liés au manque d'uniformité granulométrique de ces poudres. Un inconvénient majeur de cette non-uniformité granulométrique des matières premières en poudre est la présence de quantités parfois importantes de particules très fines qui entraînent très fréquemment des problèmes de sécurité et d'hygiène du fait du risque de pollution atmosphérique par ces très fines particules et de leur dépôt sur les murs des ateliers.

C'est le cas notamment des matières premières toxiques, ou des colorants tels que ceux utilisés en cosmétique pour la teinture des cheveux et de la peau (maquillage).

De plus, ces matières premières pulvérulentes s'écoulent généralement difficilement, ce qui rend leur manipulation malaisée et en particulier leur pesée lors d'utilisations ultérieures.

Enfin, ces matières premières pulvérulentes nécessitent souvent un temps de dissolution relativement long lorsqu'elles sont utilisées pour la fabrication de solutions finales.

En conséquence, il serait souhaitable d'obtenir des matières premières, telles que des matières colorantes, sous forme de granulés, c'est-à-dire d'agglomérats de grains de poudre ayant une forme et une surface régulières et de taille calibrée. Ces granulés, moins volatils que les particules de poudre, présentent un risque notablement diminué de pollution atmosphérique, et par conséquent d'intoxication par inhalation par les voies respiratoires, dans le cas de matières premières toxiques.

De plus, lorsque la matière première se trouve sous forme de granulés ayant une forme et une surface régulières et une taille calibrée, elle peut être aisément manipulée notamment par une facilité d'écoulement, et en particulier il est facile d'en effectuer la pesée, automatique précise pour les utilisations ultérieures.

Enfin, en réalisant des granulés ayant une microporosité élevée, on obtient une matière première qui est plus facilement solubilisée qu'une matière première sous forme de poudre.

La lyophilisation est une technique connue d'obtention de produits anhydres qui comprend la dessiccation par sublimation d'un produit préalablement solidifié par congélation. Cette lyophilisation est utilisée pour la fabrication de produits pharmaceutiques, cosmétiques, alimentaires ou vétérinaires sous forme pulvérulente.

La demande de brevet japonais publiée JP 87 305 829 JP-A-1146901 décrit la préparation d'une poudre de chitosane par dissolution de chitosane dans un acide, mise en suspension, congélation et lyophilisation pour obtenir des granulés de chitosane. Un tel procédé ne permet pas d'obtenir des granulés de surface régulière et de taille homogène.

La demande de brevet japonais publiée JP 81 152 449 JP-A-58056669 décrit un procédé de production de poudre fine qui consiste à dissoudre une substance de support dans un solvant alcoolique, à pulvériser la solution dans une atmosphère à une température inférieure à - 40°C pour obtenir de fins granulés congelés et à sécher les granulés sous vide en les conservant à l'état congelé. Du fait que les granulés sont formés par pulvérisation au moyen d'un gaz propulseur, l'écoulement obtenu se trouve sous forme d'un filet plus ou moins continu, et il n'est pas possible d'obtenir des granulés congelés de dimension homogène.

La présente invention a donc pour objet un procédé d'obtention d'une matière première, et en particulier de matières colorantes, sous forme de granulés anhydres ayant une forme et une surface régulières et une taille calibrée.

La présente invention a également pour objet un procédé tel que défini ci-dessus qui fournit des granulés ayant une cohésion suffisante pour leurs utilisations ultérieures.

La présente invention a aussi pour objet un procédé tel que défini ci-dessus qui fournit des granulés ayant une structure microporeuse facilitant la mise en solution ultérieure du produit.

Le procédé selon la présente invention permet d'obtenir une matière première, telle qu'une matière colorante, sous forme de granulés anhydres de forme et de surface régulières et de dimension calibrée, ayant une porosité élevée et présentant ainsi une meilleure solubilisation que les poudres.

Enfin, la présente invention a pour objet l'utilisation d'une matière première sous forme de granulés anhydres dans le domaine cosmétique, capillaire, vétérinaire ou pharmaceutique.

Selon l'invention, on a mis au point un procédé d'obtention d'une matière première sous forme de granulés anhydres, microporeux, de forme et surface régulières et de taille calibrée qui consiste à :
a) obtenir une dispersion ou une solution dans un solvant ou un mélange de solvants apte à être liophilisable, d'une matière première en poudre, ladite dispersion ayant une viscosité permettant de former mécaniquement des gouttes calibrées au moyen d'un dispositif mécanique dans lequel la dispersion ou la solution s'écoule goutte à goutte;
b) former mécaniquement, à partir de ladite dispersion ou solution, des gouttes calibrées;
c) congeler les gouttes formées à l'étape (c) pour obtenir des gouttes congelées; et
d) lyophiliser les gouttes congelées de l'étape (c) pour obtenir des granulés à l'état anhydre, microporeux, ayant une surface et une forme régulières et une taille calibrée, de la matière première.

Dans la présente invention, l'expression "matière première" englobe des composés simples, des mélanges de composés, ainsi que des produits finis ou semi-finis obtenus à partir des composés simples.

Un aspect important du procédé de la présente invention concerne l'étape (b) de formation mécanique de gouttes de taille calibrée à partir de la solution ou dispersion de la matière première pulvérulente. Cette étape est une étape mécanique de mise en forme qui, contrairement à d'autres techniques telles que la pulvérisation, permet d'obtenir des gouttes de forme bien définie, généralement sphérique ou semi-sphérique, une surface régulière et une dimension calibrée.

On connaît des procédés et dispositifs de mise en forme mécanique de solution et de dispersion, parmi lesquelles on peut citer les dispositifs constitués par une simple rampe de tubes ou d'aiguilles dans lesquels la solution ou la dispersion s'écoule goutte à goutte. Ces dispositifs mécaniques de mise en forme peuvent être commandés manuellement, mécaniquement ou électriquement.

Le diamètre des gouttes formées est fonction du type d'appareillage utilisé, notamment du diamètre du tube ou de l'aiguille au niveau duquel se forme la goutte (lorsqu'on utilise une rampe de tubes ou d'aiguilles), et également de la nature des éventuels additifs ajoutés à la dispersion ou solution. En général les gouttes ont un diamètre compris entre 0,1 et 10 mm, de préférence allant de 1 à 5 mm.

Les gouttes formées peuvent être constituées de matières premières de natures chimiques différentes et former ainsi une matière première sous forme de granulés, ayant les caractéristiques de chacune des matières premières.

L'association de granulés constitués de matières premières de natures différentes permet d'obtenir un produit commercialisable réunissant les propriétés intrinsèques des différents granulés constitués des matières premières.

La matière première peut se présenter directement sous forme d'une solution ou d'une dispersion dans un solvant ou mélange de solvants convenable, et dont la viscosité peut éventuellement être ajustée par l'ajout d'un agent chimique ou par la variation d'un paramètre physique, tel que la température, la concentration en extrait sec. Si la matière première se présente sous forme de poudre anhydre il est alors nécessaire de réaliser une solution ou dispersion de cette poudre dans un solvant ou mélange de solvants convenable.

Les solutions ou dispersions de matière première pulvérulente utilisées dans le procédé selon l'invention sont obtenus simplement par mise en solution ou dispersion de la poudre dans un solvant ou un mélange de solvants apte à la lyophilisation.

Parmi les solvants aptes à la lyophilisation utilisables dans la présente invention, on peut citer l'eau, l'isopentane, le diméthylsulfoxyde, la méthylamine, l'éthylamine, la diéthylamine, la propylamine, l'acide fumarique, l'acide acétique, l'alcool t-butylique, l'alcool t-amylique, le dioxane-1,4, l'isobutane, l'oxyde d'éthylène et le cyclohexane. On recommande l'eau comme solvant sublimable.

Les solutions et dispersions utilisées dans le procédé de l'invention doivent avoir une viscosité telle qu'elles puissent être mises mécaniquement sous forme de gouttes. Par conséquent, la teneur en extrait sec de la solution ou de la dispersion doit être suffisante pour que cette solution ou cette dispersion ait une viscosité adaptée au pompage et à la formation des gouttes par l'appareil destiné à la formation de gouttes. Généralement, cette fraction d'extrait sec est comprise entre 1 et 99% en poids par rapport au poids de la solution ou de la dispersion.

En général, la solution ou la dispersion a une viscosité au plus égale à 20 Pa.s et au moins suffisante pour permettre la formation de gouttes de taille définie lors de l'étape de mise en forme mécanique. De préférence, la solution ou la dispersion a une viscosité comprise entre 0,001 et 15 Pa.s.

Il est parfois nécessaire d'ajouter un ou plusieurs additifs afin d'obtenir une viscosité qui permette de former mécaniquement des gouttes calibrées et/ou conférer une certaine cohésion ou liant aux granulés. Ces additifs sont généralement choisis parmi les gélifiants et les agents de texture connus en lyophilisation. Les agents de texture peuvent éventuellement augmenter la viscosité de la solution ou de la dispersion. De préférence, les solutions et les dispersions selon l'invention comportent à la fois un gélifiant et un agent de texture.

Parmi les gélifiants utilisables dans les solutions ou dispersions selon l'invention, on peut citer les carbomères, l'hydroxyéthylcellulose, la carboxyméthylcellulose, l'agar-agar, la gomme de xanthane, l'amidon, le polyéthylèneglycol, la polyvinylpyrrolidone, la gomme de caroube, la gomme de guar, la gélatine, la caséine, la pectine, les alginates et les carraghénates.

Parmi les agents de texture utilisables dans les solutions ou dispersions selon l'invention, on peut citer le mannitol, le glucose, le lactose, le maltose, le polyéthylèneglycol, l'amidon, la polyvinylpyrrolidone, les sels minéraux, le sorbitol et le carbopol® (polymères acryliques réticulés).

Le choix de l'additif sera bien évidemment fonction de l'utilisation ultérieure envisagée pour les granulés. La quantité d'additifs peut être aisément déterminée par l'homme du métier et est fonction de l'additif choisi et du degré de viscosité souhaité pour la solution ou la dispersion.

On peut également régler la viscosité de la solution ou de la dispersion en réglant la température de la solution ou de la dispersion lors de la mise en forme mécanique ou encore en ajustant le pourcentage d'extraits secs de la solution ou de la dispersion.

D'une manière générale la viscosité de la solution ou de la suspension peut être réglée, au choix, par une dilution, l'ajout d'un agent gélifiant, par une augmentation et par une diminution de la température.

La congélation des gouttes liquides calibrées formées à l'étape de mise en forme mécanique peut s'effectuer par tout procédé bien connu. En général la température de congélation de la solution ou de la dispersion est comprise entre 0 et - 180°C.

La forme géométrique des gouttes obtenues sera fonction des étapes b) et c) du procédé d'obtention tel que défini précédement.

Les gouttes congelées obtenues subissent ensuite une dessiccation par sublimation (lyophilisation) afin d'éliminer le solvant ou le mélange de solvants. Cette lyophilisation peut s'effectuer, par exemple, dans une chambre de lyophilisation comme cela est bien connu. Cette élimination par sublimation du solvant ou du mélange de solvants des gouttes congelées permet de conserver aux granulés formés la forme des gouttes congelées initiales, et permet ainsi d'obtenir des granulés ayant une forme et une surface régulières et une taille calibrée. D'autre part, cette lyophilisation confère également aux granulés anhydres une structure microporeuse réglée.

En général, les granulés obtenus par le procédé de l'invention ont une microporosité élevée qui est liée à la vitesse de congélation (par exemple environ 60% en volume d'air et 40% en volume d'extrait sec).

De préférence, la quantité résiduelle de solvants ou du mélange de solvants, à la fin de la lyophilisation, est inférieure à 3 % en poids par rapport au poids total du produit sec.

Cette lyophilisation est un procédé bien connu et est couramment utilisée, en particulier dans l'industrie alimentaire et l'industrie pharmaceutique.

La mise en oeuvre du procédé selon l'invention permet donc d'obtenir une matière première anhydre sous forme de granulés, microporeux, de forme et de surface régulières et de taille calibrée. En effet, l'étape de mise en forme mécanique permet d'obtenir des gouttes liquides de taille déterminée et calibrée qui sont ensuite figées par congélation. La lyophilisation permet alors de conserver la forme des gouttes congelées et d'obtenir des granulés ayant une forme et une surface régulières et une taille calibrée, ainsi qu'une structure microporeuse.

En général, les granulés obtenus par le procédé de l'invention ont un diamètre moyen allant de 0,1 à 10 mm de préférence de 1 à 5 mm.

L'écart relatif de poids entre les granulés obtenus lors de la mise en oeuvre du procédé de. l'invention dépend bien évidemment de la régularité de la dimension des gouttes de liquides obtenues lors de la mise en forme mécanique et de l'homogénéité de la dispersion. Ainsi, lorsque l'on utilise un système de rampe à tubes ou aiguilles et une dispersion homogène, on peut obtenir un écart de poids entre les granulés de l'ordre de 0,5%.

Généralement, les granulés obtenus par le procédé selon la présente invention sont suffisamment cohésifs pour résister aux manipulations. On obtient en général une bonne cohésion si la solution ou la dispersion contient une fraction soluble suffisante de la matière première, c'est à dire une fraction supérieure en général à 0,5% en poids de l'extrait sec.

Le procédé de l'invention convient particulièrement à la fabrication de matières premières pour la coloration des cheveux et/ou de la peau (maquillage), de produits cosmétiques liquides tels que shampooings, d'émulsions contenant ou non des pigments, de produits alimentaires lyophilisables présentés sous forme de granulés, et de produits pharmaceutiques lyophilisables. Le procédé de l'invention est particulièrement utile pour l'obtention de granulés de matière colorante. Les colorants appropriés sont les colorants classiquement utilisés en cosmétique pour la coloration des cheveux ou de la peau (maquillage), tels que les colorants azoïques, aromatiques, et anthraquinoniques.

Les granulés de matière première selon l'invention ont généralement une forme sphérique, semi-sphérique ou de sphères aplaties et un diamètre moyen compris entre 0,1 et 10 mm, de préférence entre 1 et 5 mm ce qui en facilite la pesée et l'utilisation. La porosité élevée de ces granulés de matière première en facilite leur dissolution pour des utilisations ultérieures.

La régularité de la taille des granulés obtenus permet de réaliser des dosages très précis des matières premières, telles que des matières colorantes.

### EXEMPLE 1

On prépare la dispersion aqueuse suivante :

| | |
|---|---|
| - Paraphénylène diamine ou 2,5-diaminobenzène (colorant) | 50 g |
| - D(-) Mannitol (texturisant) | 5 g |
| - Carboxyméthylcellulose sel de sodium (gélifiant) | 0,15 g |
| - Eau déminéralisée qsp | 100 g |

La dispersion est effectuée à l'abri de l'oxygène de l'air. Après homogénéisation, la dispersion a une viscosité de 0,29 Pa.s. Elle est mise en forme par gouttage au travers d'une rampe d'aiguilles de diamètre intérieur de 2.10⁻³ m.

Les gouttelettes liquides formées sont congelées à 218°K avant d'être lyophilisées à une température de 248°K sous une pression de 30 Pa. Le séchage dure environ 20 heures.

Les granulés obtenus ont un poids moyen de 15,5 mg et résistent à une pression de 22 kPa sans s'écraser. Ils génèrent moins de 3% de particules de diamètre inférieur à 2.10⁻³m après 5 minutes sous agitation.

### EXEMPLE 2

On utilise la dispersion aqueuse suivante :

| | |
|---|---|
| - Hydroxyanthraquinoneaminopropyl méthyl morpholinium | 0,007 g |
| - Composés huileux | 19 g |
| - Conservateur | 0,4 g |
| - Amidon de maïs | 3 g |
| - Eau déminéralisée qsp | 100 g |

La viscosité de cette émulsion est suffisante pour assurer la mise en forme par gouttage au travers d'une rampe d'aiguilles de diamètre intérieur de 2.10⁻³ m sans ajout supplémentaire d'agent de texture et/ou d'agent gélifiant.

Les gouttelettes liquides sont congelées à 218°K avant d'être lyophilisées à une température de 253°K sous une pression de 30 Pa. Le séchage dure environ 24 heures.

Les granulés obtenus ont la forme de sphères aplaties et ont un poids moyen de 20 mg.

## Revendications

1. Procédé d'obtention d'une matière première sous forme de granulés à l'état anhydre, microporeux, ayant une surface et une forme régulières et une taille calibrée, caractérisé en ce qu'il consiste à :
(a) obtenir une dispersion ou une solution dans un solvant ou un mélange de solvants lyophilisable, d'une matière première, ladite dispersion ou solution ayant une viscosité permettant de former mécaniquement des gouttes calibrées;
(b) former mécaniquement, à partir de ladite dispersion ou solution, des gouttes calibrées, au moyen d'un dispositif mécanique dans lequel la dispersion ou la solution s'écoule goutte à goutte;
(c) congeler les gouttes formées à l étape (b) pour obtenir des gouttes congelées; et
(d) lyophiliser les gouttes congelées obtenues à l'étape (c) pour obtenir des granulés à l'état anhydre, microporeux, ayant une surface et une forme régulières, et une taille calibrée, de la matière première.

2. Procédé selon la revendication 1, caractérisé en ce que la solution ou la dispersion a une viscosité au plus égale à 20 Pa.s.

3. Procédé selon la revendication 2, caractérisé en ce que la dispersion ou la solution a une viscosité comprise entre 0,001 et 15 Pa.s.

4. Procédé selon l'une quelconque des revendications 1 à 3, caractérisé en ce que dans l'étape (a) on ajuste la viscosité de la dispersion ou de la solution au choix par une dilution, l'ajout d'un agent gélifiant, par une augmentation, et une diminution de la température.

5. Procédé selon l'une quelconque des revendications 1 à 4, caractérisé en ce qu'on ajoute un agent gélifiant choisi parmi les carbomères, l'hydroxyéthylcellulose, la carboxyméthylcellulose, l'agar-agar, la gomme de xanthane, l'amidon, le polyéthylèneglycol, la polyvinylpyrrolidone, la gomme de caroube, la gomme de guar, la gélatine, la caséine, la pectine, les alginates, et les carraghénates.

6. Procédé selon l'une quelconque des revendications 1 à 5, caractérisé en ce qu'on ajoute un agent de texture.

7. Procédé selon la revendication 6, caractérisé en ce que l'agent de texture est choisi parmi le mannitol, le maltose, le glucose, le lactose, le polyéthylèneglycol, l'amidon, la polyvinylpyrrolidone, les sels minéraux, le sorbitol et un polymère acrylique réticulé.

8. Procédé selon l'une quelconque des revendications précédentes caractérisé en ce que les granulés obtenus sont sous forme sphérique, semi-sphérique ou de sphères aplaties.

9. Procédé selon l'une quelconque des revendications précédentes, caractérisé en ce que les granulés obtenus ont un diamètre moyen compris entre 0,1 et 10 mm et de préférence entre 1 et 5 mm.

10. Procédé selon l'une quelconque des revendications précédentes, caractérisé en ce que la matière première est une matière colorante.

## Claims

1. Process for producing a starting material in the form of granules in the microporous anhydrous state having an even surface, a regular shape and a graded size, characterized in that it consists in:
(a) obtaining a dispersion or a solution, in a lyophilizable solvent or mixture of solvents, of a starting material, the said dispersion or solution having a viscosity which makes it possible mechanically to form graded drops;
(b) mechanically forming graded drops from the said dispersion or solution , by means of a mechanical device in which the dispersion or the solution flows dropwise;
(c) freezing the drops formed in Stage (b) in order to obtain frozen drops; and
(d) lyophilizing the frozen drops obtained in Stage (c) in order to obtain granules of the starting material in the microporous anhydrous state having an even surface, a regular shape and a graded size.

2. Process according to Claim 1, characterized in that the solution or the dispersion has a viscosity not greater than 20 Pa·s.

3. Process according to Claim 2, characterized in that the dispersion or the solution has a viscosity of between 0.001 and 15 Pa·s.

4. Process according to any one of Claims 1 to 3, characterized in that, in Stage (a), the viscosity of the dispersion or of the solution is adjusted, as desired, by a dilution, the addition of a gelling agent or by an increase and a reduction in the temperature.

5. Process according to any one of Claims 1 to 4, characterized in that the addition is carried out of a gelling agent chosen from carbomers, hydroxyethyl cellulose, carboxymethyl cellulose, agar, xanthan gum, starch, polyethylene glycol, polyvinylpyrrolidone, locust bean gum, guar gum, gelatin, casein, pectin, alginates and carrageenates.

6. Process according to any one of Claims 1 to 5, characterized in that a structuring agent is added.

7. Process according to Claim 6, characterized in that the structuring agent is chosen from mannitol, maltose, glucose, lactose, polyethylene glycol, starch, polyvinylpyrrolidone, inorganic salts, sorbitol and a crosslinked acrylic polymer.

8. Process according to any one of the preceding claims, characterized in that the granules obtained are in a spherical or semi-spherical shape or in the shape of flattened spheres.

9. Process according to any one of the preceding claims, characterized in that the granules obtained have a mean diameter of between 0.1 and 10 mm and preferably between 1 and 5 mm.

10. Process according to any one of the preceding claims, characterized in that the starting material is a colouring material.

## Patentansprüche

1. Verfahren zum Erhalt eines Ausgangsstoffes in Form mikroporöser Granulate in wasserfreiem Zustand, welche eine regelmäßige Oberfläche und Form und eine geeichte Größe aufweisen,
dadurch **gekennzeichnet**, daß man:
a) eine lyophilisierbare Dispersion oder Lösung eines Ausgangsstoffes in einem Lösungsmittel oder einer Mischung von Lösungsmitteln erhält, wobei die genannte Dispersion oder Lösung eine Viskosität aufweist, die es ermöglicht, geeichte Tropfen mechanisch zu bilden,
b) aus der genannten Dispersion oder Lösung geeichte Tropfen mittels einer mechanischen Vorrichtung mechanisch bildet, worin sich die Dispersion oder Lösung tropfenweise verteilt,
c) die in Stufe (b) gebildeten Tropfen gefriert, um gefrorene Tropfen zu erhalten, und man
d) die in Stufe (c) erhaltenen gefrorenen Tropfen lyophilisiert, um mikroporöse Granulate des Ausgangsstoffes in wasserfreiem Zustand zu erhalten, welche eine regelmäßige Oberfläche und Form und eine geeichte Größe aufweisen.

2. Verfahren gemäß Anspruch 1,
dadurch **gekennzeichnet**, daß
die Lösung oder die Dispersion eine Viskosität von höchstens 20 Pa x s aufweisen.

3. Verfahren gemäß Anspruch 2,
dadurch **gekennzeichnet**, daß
die Dispersion oder die Lösung eine Viskosität von 0,001 bis 15 Pa x s aufweisen.

4. Verfahren gemäß einem der Ansprüche 1 bis 3,
dadurch **gekennzeichnet**, daß
man in der Stufe (a) die Viskosität der Dispersion oder der Lösung wahlweise durch einen Verdünnungsvorgang, die Zugabe eines Geliermittels, durch eine Erhöhung und eine Absenkung der Temperatur einstellt.

5. Verfahren gemäß einem der Ansprüche 1 bis 4,
dadurch **gekennzeichnet**, daß
man ein Geliermittel zufügt, ausgewählt aus Carbomeren, Hydroxyethylcellulose, Carboxymethylcellulose, Agar-Agar, Xanthan-Gummi, Stärke, Polyethylenglycol, Polyvinylpyrrolidon, Johannisbrot-Gummi, Guar-gummi, Gelatine, Kasein, Pektin, Alginaten und aus Karraghenaten.

6. Verfahren gemäß einem der Ansprüche 1 bis 5,
dadurch **gekennzeichnet**, daß
man ein Texturiermittel zufügt.

7. Verfahren gemäß Anspruch 6,
dadurch **gekennzeichnet**, daß
das Texturiermittel aus Mannit, Maltose, Glucose, Lactose, Polyethylenglycol, Stärke, Polyvinylpyrrolidon, Mineralsalzen, Sorbit und aus aus einem vernetzten Acrylpolymer ausgewählt ist.

8. Verfahren gemäß einem der vorhergehenden Ansprüche,
dadurch **gekennzeichnet**, daß
die erhaltenen Granulate in kugelförmiger, halb-kugelförmiger oder in Form abgeflachter Kugeln vorliegen.

9. Verfahren gemäß einem der vorhergehenden Ansprüche,
dadurch **gekennzeichnet**, daß
die erhaltenen Granulate einen mittleren Durchmesser von 0,1 bis 10 und vorzugsweise von 1 bis 5 mm aufweisen.

10. Verfahren gemäß einem der vorhergehenden Ansprüche,
dadurch **gekennzeichnet**, daß
der Ausgangsstoff ein Farbstoff ist.
